# EUROPEAN PATENT APPLICATION

(11) **EP 4 814 100 A1**
(43) Date of publication of application: **30.09.2026**
(21) Application number: 26166812.3
(22) Date of filing: 23.03.2026
(51) Int. Cl.: C12M 1/42, C12M 1/36

(54) **CELL PUNCTURE DEVICE, CELL PUNCTURE METHOD, AND NON- TRANSITORY COMPUTER READABLE MEDIUM**

(30) Priority: 28.03.2025 JP 2025057046
(71) Applicant: Yokogawa Electric Corporation, Musashino-shi, Tokyo 180-8750 (JP)
(72) Inventor: KUWATA, Masahiro, Musashino-shi, Tokyo, 180-8750 (JP)
(74) Representative: Stöckeler, Ferdinand

(57) **Abstract**

A cell puncture device 10 according to the present disclosure includes a needle 17a that punctures a cell S along a puncture direction, a driver 16 that drives the needle 17a, and a controller 51 that controls operations of the driver 16. The controller 51 performs a first operation in which the driver 16 is moved in the puncture direction to puncture the cell S with the needle 17a, and a second operation in which the driver 16 is moved in the puncture direction or in a withdrawal direction opposite the puncture direction to reduce vibration occurring within the cell puncture device 10 due to the first operation.

## Description

### CROSS-REFERENCE TO RELATED APPLICATION

This application claims priority to Japanese Patent Application No. 2025-057046, filed on March 28, 2025, the entire contents of which are incorporated herein by reference.

### TECHNICAL FIELD

The present disclosure relates to a cell puncture device, a cell puncture method, and a non-transitory computer readable medium.

### BACKGROUND

In conventional research and applied development related to cells, technology for puncturing cells with needles is known, in order to accurately inject chemical solutions or the like into specific cells as samples, or to suck substances inside cells. For example, Patent Literature (PTL) 1 discloses that, in a device that uses a multi-barrel nano-pipette with at least two electrodes inside multiple barrels, one barrel draws out a cell inclusion and the other injects a substance into a cell.

### CITATION LIST

### Patent Literature

PTL 1: JP 6453300 B2

### SUMMARY

In the conventional technology described in PTL 1, for example, when a needle punctures a cell, a needle tip may vibrate inside a cell wall and cause damage to the interior of the cell wall. When vibration is large, it is assumed that the needle is repeatedly inserted into and removed from the cell, which also causes damage to the cell.

The present disclosure aims to provide a cell puncture device, a cell puncture method, and a non-transitory computer readable medium that can suppress vibration of a needle.
[1] A cell puncture device, including:
   a needle configured to puncture a cell along a puncture direction;
   a driver configured to drive the needle; and
   a controller configured to control operations of the driver,
   wherein the controller is configured to perform:
      a first operation in which the driver is moved in the puncture direction to puncture the cell with the needle; and
      a second operation in which the driver is moved in the puncture direction or in a withdrawal direction opposite the puncture direction to reduce vibration occurring within the cell puncture device due to the first operation.
   Such a cell puncture device can suppress vibration of the needle.
**[2] In** the cell puncture device according to [1] above, the controller may perform the second operation before the first operation.
   This can suppress vibration of the needle during the first operation.
**[3] In** the cell puncture device according to [2] above, the amount of movement of the driver in the second operation may be greater than or equal to the amount of movement of the driver in the first operation.
   This can effectively suppress vibration of the needle during the first operation.
[4] **In** the cell puncture device according to [2] above, the amount of movement of the driver in the second operation may be less than the amount of movement of the driver in the first operation.
   This facilitates puncturing the cell with the needle.
**[5] In** the cell puncture device according to any one of [1] to [4] above, when the driver is moved in the withdrawal direction in the second operation, the controller may be configured to further perform, between the first operation and the second operation, a third operation in which the driver is moved to an opposite direction from a preceding operation at a lower speed than in the first and second operations.
   This allows the position of the driver that has been moved in the preceding operation to be closer to its position prior to the preceding operation, thus facilitating performing a subsequent operation, among the first and second operations, that is performed after the third operation.
**[6] In** the cell puncture device according to [5] above, the amount of movement of the driver in the third operation may be greater than or equal to the amount of movement of the driver in the preceding operation, among the first and second operations, that is performed before the third operation.
   This allows the amount of movement of the driver in the preceding operation to be greater than the amount of movement of the driver in the subsequent operation, thus effectively suppressing vibration of the needle even when vibration that has occurred during the preceding operation is attenuated between the preceding operation and the subsequent operation.
[7] The cell puncture device according to any one of [1] to [6] above may include:
   a base; and
   an arm disposed movably with respect to the base, the arm being configured to support the driver.
   This allows the position of the driver relative to the base to be easily adjusted by the arm.
[8] In the cell puncture device according to [7] above, the controller may be configured to calculate a time interval between the start time of the first operation and the start time of the second operation, according to the linear distance or the path distance between the base and the driver supported by the arm.
   This allows the first and second operations to be performed based on the calculated time interval, resulting in effectively suppressing vibration of the needle.
[9] The cell puncture device according to [7] above may include a fixed part disposed with respect to an imager configured to image the cell. The controller may be configured to calculate a time interval between the start time of the first operation and the start time of the second operation, according to the linear distance or the path distance between the fixed part and the driver supported by the arm.
   This allows the first and second operations to be performed based on the calculated time interval, resulting in effectively suppressing vibration of the needle.
[10] The cell puncture device according to any one of [7] to [9] above may include a sensor unit disposed on the arm, the sensor unit being configured to detect the vibration state of the arm. The controller may be configured to calculate a period of natural vibration of the arm from a vibration value of the arm detected by the sensor unit, and calculate a time interval between the start time of the first operation and the start time of the second operation according to the period.
   This allows the first and second operations to be performed based on the calculated time interval, resulting in effectively suppressing vibration of the needle.
[11] In the cell puncture device according to any one of [8] to [10] above,
   when the driver is moved in the withdrawal direction in the second operation, the following condition may be satisfied: $0.9 \times m \times p \leq t \leq 1.1 \times m \times p$
   m: an integer greater than or equal to 1,
   p: a period of natural vibration of the arm,
   t: the time interval
   when the driver is moved in the puncture direction in the second operation, the following condition may be satisfied: $0.9 \times \left(2m-1\right) \times p / 2 \leq t \leq 1.1 \times \left(2m-1\right) \times p / 2$
   m: an integer greater than or equal to 1,
   p: a period of natural vibration of the arm,
   t: the time interval.
   This can effectively suppress vibration of the needle.
[12] In the cell puncture device according to [10] or [11] above, the period of the natural vibration of the arm may be a period of the arm when only the arm is vibrating, or a period of the arm when the arm is vibrating together with another component of the cell puncture device.
   This can effectively suppress vibration of the needle.
[13] In the cell puncture device according to any one of [1] to [12] above, the controller may be configured to move the driver such that the movement speed of the needle in the first operation is 5 m/s or more.
   This allows the needle to puncture the cell at a speed sufficient for the needle to penetrate the cell wall.
[14] A cell puncture method using a cell puncture device including a needle configured to puncture a cell along a puncture direction, and a driver configured to drive the needle, the cell puncture method including:
   a first operation in which the driver is moved in the puncture direction to puncture the cell with the needle; and
   a second operation in which the driver is moved in the puncture direction or in a withdrawal direction opposite the puncture direction to reduce vibration occurring within the cell puncture device due to the first operation.
   Such a cell puncture method can suppress vibration of the needle.
[15] A non-transitory computer readable medium storing a program executable by one or more processors, the program configured to cause a cell puncture device to execute operations, the cell puncture device including a needle configured to puncture a cell along a puncture direction, and a driver configured to drive the needle, the operations including:
   a first operation in which the driver is moved in the puncture direction to puncture the cell with the needle; and
   a second operation in which the driver is moved in the puncture direction or in a withdrawal direction opposite the puncture direction to reduce vibration occurring within the cell puncture device due to the first operation.
   Such a program can suppress vibration of the needle.

According to the present disclosure, it is possible to provide a cell puncture device, a cell puncture method, and a non-transitory computer readable medium that can suppress vibration of a needle.

### BRIEF DESCRIPTION OF THE DRAWINGS

In the accompanying drawings:
FIG. 1 is a schematic diagram illustrating an example of a configuration of a microscope system with a cell puncture device according to an embodiment of the present disclosure;
FIG. 2 is a flowchart illustrating an example of operations of the cell puncture device in FIG. 1;
FIG. 3 is a graph illustrating a simulation result of a comparative example;
FIG. 4 is a graph illustrating variations over time for a longer period than in FIG. 3;
FIG. 5 is a graph illustrating a simulation result of a first example;
FIG. 6 is a graph illustrating variations over time for a longer period than in FIG. 5;
FIG. 7 is a graph illustrating a simulation result of a second example;
FIG. 8 is a graph illustrating variations over time for a longer period than in FIG. 7;
FIG. 9 is a graph illustrating a simulation result of a third example;
FIG. 10 is a graph illustrating variations over time for a longer period than in FIG. 9;
FIG. 11 is a graph illustrating a simulation result of a fourth example;
FIG. 12 is a graph illustrating variations over time for a longer period than in FIG. 11;
FIG. 13 is a graph illustrating a simulation result of a fifth example;
FIG. 14 is a graph illustrating variations over time for a longer period than in FIG. 13;
FIG. 15 is a graph illustrating a simulation result of a sixth example;
FIG. 16 is a graph illustrating variations over time for a longer period than in FIG. 15;
FIG. 17 is a graph illustrating a simulation result of a seventh example; and
FIG. 18 is a graph illustrating variations over time for a longer period than in FIG. 17.

### DETAILED DESCRIPTION

### (Background and Problems)

The background and problems of conventional technology will be described in more detail.

In recent years, in research into biological systems and the like, there have been research into elucidating cell functions by injecting specific chemical solutions into cells and observing changes in the cells, and research into causing specific modifications to occur in specific cells by injecting, into the cells, chemical solutions to modify genes. In addition, there has also been applied development aiming at application to the production of chemical solutions or other substances. On the other hand, there have also been research into elucidating cell functions and applied development aiming at application to production by sucking and recovering, from specific cells, some of components that constitute the cells. In the above research and applied development, it is required, for example, to accurately inject the chemical solutions into the specific cells or to accurately suck the components from the specific cells, so it is desired that cell puncture devices can precisely puncture cells with needles.

PTL 1 discloses conventional technology for a method and device of injecting a chemical solution into a cell by controlling the position of a fine needle using a piezoelectric element, puncturing the cell by a needle tip, and controlling a voltage. Similarly, conventional technology for a method and device for sucking a substance from the interior of the cell is also disclosed.

In general, cells protect their interiors by cell walls, which are located in more external parts of the cells and cover the interiors. Therefore, in order to inject a chemical solution into a cell or to take out a substance from the interior of a cell by suction, a cell puncture device needs to insert a needle into the interior of the cell by controlling the position and speed of a needle tip so that the needle tip penetrates the cell wall. For this purpose, the cell puncture device preferably moves the needle at a high speed when the needle punctures the cell.

For example, in the case of a certain animal cell with a soft cell wall, the needle can penetrate the cell wall even if the cell puncture device moves the needle at a low speed when the needle punctures the cell. On the other hand, in the case of a certain plant cell with a hard cell wall, the needle cannot penetrate the cell wall unless the cell puncture device moves the needle at a high speed when the needle punctures the cell, which causes the problem of difficulty in inserting the needle tip into the cell.

As a method of moving the needle at a high speed as described above, a method of moving the needle using a piezoelectric element included in a driver that drives the needle is known. The piezoelectric element can cause an electrostrictive effect to occur inside itself by application of a voltage, and can expand and contract the piezoelectric element itself. The expansion and contraction of the piezoelectric element can respond more quickly than actuators such as general motors. Therefore, with the use of the piezoelectric element, the cell puncture device can cause the needle to puncture the cell at a high speed.

However, due to the high-speed movement of the needle using the piezoelectric element, there is a problem that the needle vibrates based on a reaction force when the needle is driven by the driver. For example, when part of the driver is moved to puncture the cell, a reaction force occurs on a fixed side of the driver and causes vibration. In addition, when the needle punctures the cell, a force due to reaction is applied to a structure that supports the piezoelectric element. This causes the entire structure to move in the opposite direction to a puncture direction of the needle. As a result, there is also a problem that the needle vibrates after the puncture. When the needle vibrates after the puncture, the needle tip may vibrate inside the cell wall, and cause damage to the interior of the cell wall. When vibration is large, it is assumed that the needle is repeatedly inserted into and removed from the cell, which also causes damage to the cell. Therefore, for example, when a chemical solution is injected into the interior of the cell and its progress is observed, a cell survival rate is reduced due to damage to the cell caused by the vibration of the needle tip. As a result, there are cases in which the effects of the injected chemical solution cannot be evaluated.

In order to solve the problems described above, the present disclosure aims to provide a cell puncture device, a cell puncture method, and a non-transitory computer readable medium that can suppress vibration of a needle.

An embodiment of the present disclosure will be mainly described below with reference to the attached drawings. In the following description, x-, y-, and z-directions are with respect to the directions of the arrows in the drawings.

### (Configurations of Cell Puncture Device 10 and Microscope System 1)

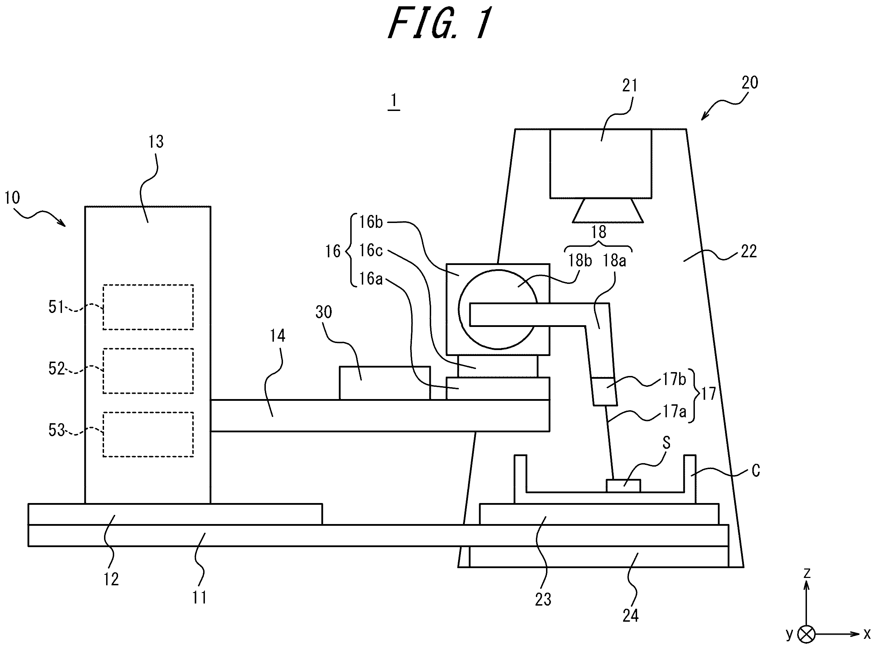

FIG. 1 is a schematic diagram illustrating an example of a configuration of a microscope system 1 with a cell puncture device 10 according to the embodiment of the present disclosure. With reference to FIG. 1, an example of the configuration and functions of the microscope system 1 with the cell puncture device 10 according to the present embodiment will be mainly described. The microscope system 1 has the cell puncture device 10 and a microscope 20 that images a cell S to be punctured by a needle 17a of the cell puncture device 10.

The microscope 20 includes any microscope that can image the cell S. The microscope 20 includes, for example, a confocal microscope. The microscope 20 has any camera 21 that can image the cell S. The camera 21 constitutes an imager of the microscope 20. The microscope 20 has a pillar 22 that locates the camera 21 on one side of the cell S in the z-direction, so that the camera 21 can image the cell S from that side. The pillar 22 supports the camera 21 connected to an end of the pillar 22 on that side. The microscope 20 has a holder 23 that holds a petri dish C, on which the cell S is disposed, from the other side in the z-direction. The holder 23 is configured as a stage that is movable in two directions, the x- and y-directions. The microscope 20 has a support 24 that is located at an end of the pillar 22 on the other side and supports the holder 23, which holds the petri dish C.

The cell puncture device 10 has a first fixed part 11 that is disposed with respect to the imager, which images the cell S. The first fixed part 11 is fixed to, for example, the microscope 20, which images the cell S. The first fixed part 11 is configured in the shape of an arm and extends in the x-direction. The first fixed part 11 is disposed with respect to the holder 23 and the support 24 and is fixed to the microscope 20 by screwing one side of the first fixed part 11 in the x-direction onto the support 24 of the microscope 20. As an example, the first fixed part 11 is located between the holder 23 and the support 24, but the present disclosure is not limited thereto. The holder 23 may be located under the first fixed part 11. The first fixed part 11 is not limited to being screwed onto the support 24, but may be fixed to the microscope 20 in any other manner, such as by joining, fitting, or engaging. The cell puncture device 10 can be attached to the microscope 20 via the first fixed part 11.

The cell puncture device 10 has a base 12 that is connected to the other side of the first fixed part 11 in the x-direction and located on a surface of the first fixed part 11. The cell puncture device 10 has a first driver 13 that is supported by the first fixed part 11 and the base 12 so as to protrude from the base 12 to the positive side in the z-direction. The cell puncture device 10 has an arm 14 that extends from the first driver 13 to the positive side in the x-direction. The arm 14 is disposed with respect to the first fixed part 11 and the base 12. For example, the arm 14 is disposed in parallel with the first fixed part 11 and the base 12. The first driver 13 drives the arm 14 so that the arm 14 is movable in each of the x-, y-, and z-directions with respect to the first fixed part 11 and the base 12. For example, the first driver 13 may include an actuator that includes a ball screw and a motor to drive the arm 14.

The cell puncture device 10 has a second driver 16 supported by the arm 14. The second driver 16 is located on a surface of the arm 14 on the positive side in the z-direction. However, the disposition of the second driver 16 is not limited thereto. The second driver 16 may not be located on the surface of the arm 14 on the positive side in the z-direction. For example, the second driver 16 may be located on a surface of the arm 14 on the negative side in the z-direction. The second driver 16 may be located on a surface of the arm 14 in the x-direction, or may be located on a surface inclined with respect to the x- and z-directions.

The second driver 16 needs to move the needle 17a at a high speed in order to puncture the cell S. In order to achieve such high-speed movement of the needle 17a, the second driver 16 includes, for example, a piezoelectric element 16c that drives the needle 17a. The second driver 16 includes a second fixed part 16a connected to the arm 14, the piezoelectric element 16c that is connected to the second fixed part 16a and drives the needle 17a, and a movable part 16b connected to the piezoelectric element 16c. The movable part 16b is movable relative to the second fixed part 16a. The piezoelectric element 16c interposed between the movable part 16b and the second fixed part 16a drives the needle 17a so that, for example, a needle tip of the needle 17a moves along the z-direction. The movable part 16b causes the needle 17a to puncture the cell S by moving relative to the second fixed part 16a.

The cell puncture device 10 has a needle unit 17 that disposes, at a tip end, the needle 17a to puncture the cell S and that is driven by the movable part 16b of the second driver 16. The needle unit 17 has the needle 17a that is driven by the second driver 16 to puncture the cell S, and a needle fixing part 17b that fixes the needle 17a.

The cell puncture device 10 has a needle support 18 that is connected to the needle fixing part 17b to dispose the needle unit 17 at a distal end. The needle support 18 has a support head 18a connected to the needle fixing part 17b, and a support head fixing part 18b connected to the support head 18a. The needle unit 17 can be operated by the second driver 16, by being supported by the support head 18a and by the support head 18a being attached to the support head fixing part 18b.

The needle 17a is driven by the second driver 16 to puncture the cell S along the puncture direction. In the present disclosure, the "puncture direction" refers to, for example, the direction from the positive size to the negative side in the z-direction.

The cell puncture device 10 may be configured so that by moving the base 12 in the x- or y-direction or rotating the base 12 relative to the first fixed part 11, the entire configuration including the first driver 13, the arm 14, the second driver 16, and the needle 17a can be withdrawn from the microscope 20. The movement of the base 12 in the x-direction or in the y-direction can be easily achieved, for example, by disposing linear guides, cross roller guides, and the like between the first fixed part 11 and the base 12. The rotation of the base 12 can be easily achieved, for example, by disposing ball bearings, cross roller bearings, and the like between the first fixed part 11 and the base 12.

The cell puncture device 10 may further have a fixing part (not illustrated in the drawings) between the first fixed part 11 and the base 12 in order to fix the relative movement of the base 12 in the x- or y-direction or the relative rotation of the base 12 with respect to the first fixed part 11. For example, the fixing part may fix the base 12 using a frictional force of a pin that can move in the z-direction so as to be pressed against the first fixed part 11 by a spring, or may fix the base 12 by a similar pin being engaged in a groove provided in the first fixed part 11. The pin can be easily lifted by disposing an operation unit to lift the pin to the positive side in the z-direction. The pin allows the base 12 to be moved and fixed relative to the first fixed part 11.

The cell puncture device 10 can accurately dispose the base 12 with respect to the first fixed part 11, due to the above-described configuration. The cell puncture device 10 can reduce fluctuations in the position of the needle 17a due to play of the base 12 with respect to the first fixed part 11.

The cell puncture device 10 has a controller 51 that controls operations of the second driver 16. The controller 51 is disposed within the first driver 13, but the present disclosure is not limited thereto. The controller 51 may not be disposed within the first driver 13. For example, the controller 51 may be disposed outside the first driver 13, i.e., in a portion other than the first driver 13.

The controller 11 includes at least one processor. In the present disclosure, the "processor" is a general purpose processor or a dedicated processor specialized for particular processing, but is not limited thereto. The controller 51 includes, for example, a Central Processing Unit (CPU).

The cell puncture device 10 has a sensor unit 30 that is disposed on the arm 14 to detect the vibration state of the arm 14. The sensor unit 30 includes, for example, a strain gauge that electrically detects strain in the arm 14. When the sensor unit 30 includes a strain gauge, the controller 51 may calculate the position or speed of the arm 14 from the amount of strain in the arm 14 and calculate the period of the natural vibration of the arm 14. The sensor unit 30 may include an accelerometer that measures the acceleration of the arm 14. When the sensor unit 30 includes an accelerometer, the controller 51 may calculate the position or speed of the arm 14 from the integral of the acceleration of the arm 14 and calculate the period of the natural vibration of the arm 14.

The sensor unit 30 is disposed on the positive side of the arm 14 in the z-direction. The sensor unit 30 is located, in the arm 14, on the negative side of the second driver 16 in the x-direction. However, the disposition of the sensor unit 30 is not limited thereto. For example, the sensor unit 30 may be disposed on the negative side of the arm 14 in the z-direction. For example, the sensor unit 30 may be disposed, in the arm 14, on the positive side of the second driver 16 in the x-direction.

The cell puncture device 10 has a detector 52 that detects the position of the arm 14. In the present disclosure, the "position of the arm 14" is, for example, the position of a distal end of the arm 14. The detector 52 includes, for example, limit sensors that are disposed at extended and retracted positions of the arm 14. In this case, the controller 51 may calculate the position of the arm 14 by counting the number of pulses since the arm 14 has passed a limit sensor. The detector 52 may include an encoder that measures the amount of movement of the arm 14 and/or a rotation sensor that measures the absolute number of revolutions of a motor included in the first driver 13, which drives the arm 14. In this case, the controller 51 may calculate the position of the arm 14 based on measurement values of the encoder and/or the rotation sensor.

The detector 52 is disposed within the first driver 13. However, the disposition of the detector 52 is not limited thereto. The detector 52 may be disposed outside of the first driver 13.

The cell puncture device 10 has a memory 53. The memory 53 includes a volatile memory such as a random access memory (RAM). The memory 53 functions, for example, as a cache memory. The memory 53 is disposed within the first driver 13. However, the memory 53 may be disposed outside the first driver 13. The memory 53 temporarily stores, for example, information obtained by operations of the cell puncture device 10.

### (Cellular Puncture Method)

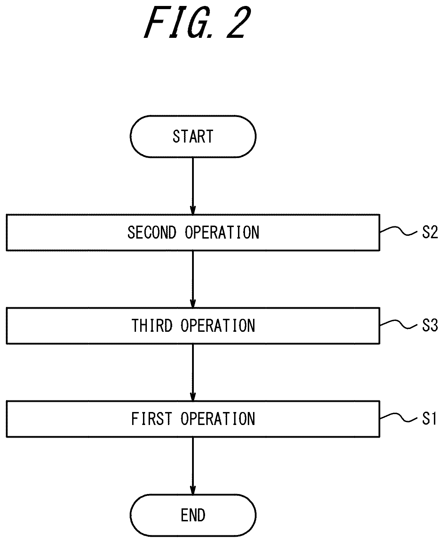

FIG. 2 is a flowchart illustrating an example of a cell puncture method performed by the cell puncture device 10 in FIG. 1. The flowchart in FIG. 2 illustrates a basic processing flow performed by the controller 51 of the cell puncture device 10. With reference to FIG. 2, an example of the cell puncture method performed by the cell puncture device 10 in FIG. 1 will be mainly described.

As illustrated in FIG. 2, the cell puncture method in this example includes a second operation S2, a third operation S3, and a first operation S1. In the cell puncture method of this example, the controller 51 performs the second operation S2, the third operation S3, and the first operation S1 in this order.

In the second operation S2, the controller 51 moves the second driver 16 in a withdrawal direction opposite the puncture direction, to reduce vibration occurring within the cell puncture device 10 due to the first operation S1. In the present disclosure, the "withdrawal direction" is, for example, the direction from the negative side to the positive side in the z-direction. Specifically, in the second operation S2 of this example, the controller 51 moves the movable part 16b in the withdrawal direction with respect to the second fixed part 16a by expanding or contracting the piezoelectric element 16c. The controller 51 may move the second driver 16 so that the movement speed of the needle 17a in the second operation S2 coincides or approximately coincides with the movement speed of the needle 17a in the first operation S1 to be performed thereafter.

The amount of movement of the second driver 16 in the second operation S2 is greater than or equal to the amount of movement of the second driver 16 in the first operation S1. Specifically, in the second operation S2 of this example, the controller 51 moves the movable part 16b of the second driver 16 in the withdrawal direction with respect to the second fixed part 16a by an amount greater than or equal to the amount of movement of the movable part 16b in the puncture direction with respect to the second fixed part 16a in the first operation S1.

In the third operation S3, the controller 51 moves the second driver 16 at a lower speed than the speed at which the second driver 16 has been moved in the second operation S2, in the puncture direction opposite the withdrawal direction in which the second driver 16 has been moved in the second operation S2. Specifically, in the third operation S3 of this example, by expanding or contracting the piezoelectric element 16c, the controller 51 moves the movable part 16b in the puncture direction with respect to the second fixed part 16a at a lower speed than the speed at which the movable part 16b has been moved in the second operation S2.

The amount of movement of the second driver 16 in the third operation S3 is greater than or equal to the amount of movement of the second driver 16 in the second operation S2 that has been performed before the third operation S3. Specifically, in the third operation S3 of this example, the controller 51 moves the movable part 16b in the puncture direction with respect to the second fixed part 16a by an amount greater than or equal to the amount of movement of the movable part 16b in the withdrawal direction with respect to the second fixed part 16a in the second operation S2.

In the first operation S1, the controller 51 moves the second driver 16 in the puncture direction to puncture the cell S with the needle 17a. In the first operation S1 of this example, the controller 51 moves the movable part 16b in the puncture direction with respect to the second fixed part 16a by expanding or contracting the piezoelectric element 16c.

The controller 51 calculates the time interval between the start time of the first operation S1 and the start time of the second operation S2 (hereinafter simply referred to as "time interval") according to the linear distance or path distance between the base 12 and the second driver 16 supported by the arm 14. The controller 51 then performs the first operation S1 and the second operation S2 based on the calculated time interval. In the present disclosure, the "linear distance" is, for example, a distance when the base 12 and the second driver 16 are virtually connected by a straight line. In the present disclosure, the "path distance" is, for example, the total length of a plurality of straight lines when the base 12 and the second driver 16 are connected by the plurality of straight lines extending in the x-, y-, and z-axes along the components of the cell puncture device 10. The controller 51 may calculate the linear distance or path distance based on the detection results of the detector 52.

The controller 51 may calculate the period of the natural vibration of the arm 14 from a vibration value of the arm 14 detected by the sensor unit 30, and calculate the time interval between the start time of the first operation S1 and the start time of the second operation S2 according to the period. The controller 51 may calculate the time interval using the sensor unit 30 while the puncture operation to the cell S is actually being performed, or may perform the puncture operation in advance before the puncture operation to the cell S is actually performed and calculate the time interval based on the period of the natural vibration of the arm 14 calculated from the detection results of the sensor unit 30 at that time.

The controller 51 may calculate the time interval based on the period of the natural vibration of the arm 14 to satisfy the following equation: $0.9 \times m \times p \leq t \leq 1.1 \times m \times p$
m: an integer greater than or equal to 1,
p: the period of the natural vibration of the arm 14,
t: the time interval.

The controller 51 may calculate the time interval using reference values for the time interval stored in advance in the memory 53 or the like. Specifically, for example, the controller 51 may calculate, based on a time interval t1 when the arm 14 is extended in the x-direction (when the arm 14 is at a position x1) and a time interval t2 when the arm 14 is retracted in the x-direction (when the arm 14 is at a position x2), a time interval t when the arm 14 is at a position x therebetween by the following equation: $t = t 1 + \left(t2-t1\right) / \left(x2-x1\right) \times \left(x-x1\right)$ The time intervals t1 and t2 may be calculated in advance at the factory and stored in the memory 53.

When the arm 14 is moved in the three directions, i.e., the x-, y-, and z-directions, the controller 51 may calculate, based on a time interval tx1 when the arm 14 is at a position x1, a time interval tx2 when the arm 14 is at a position x2, a time interval ty1 when the arm 14 is at a position y1, a time interval ty2 when the arm 14 is at a position y2, a time interval tz1 when the arm 14 is at a position z1, and a time interval tz2 when the arm 14 is at a position z2, a time interval t when the arm 14 is at a position between the positions x1 and x2, between the positions y1 and y2, and between the positions z1 and z2 by the following equation: $\begin{matrix}t = \\ tx 1 + \left(tx2-tx1\right) / \left(x2-x1\right) \times \left(x-x1\right) \\ + ty 1 + \left(ty2-ty1\right) / \left(y2-y1\right) \times \left(y-y1\right) \\ + tz 1 + \left(tz2-tz1\right) / \left(z2-z1\right) \times \left(z-z1\right)\end{matrix}$

Furthermore, when a time interval t follows a certain function, the controller 51 may calculate the time interval t by the following equation: $t = Fx \left(x\right) + Fy \left(y\right) + Fz \left(z\right)$

In the first operation S1, the controller 51 moves the second driver 16 so that the movement speed of the needle 17a in the first operation S1 is 5 m/s or more. Specifically, in the first operation S1 of this example, the controller 51 moves the movable part 16b in the puncture direction with respect to the second fixed part 16a by expanding or contracting the piezoelectric element 16c so that the movement speed of the needle 17a is 5 m/s or more.

### (Effects)

The cell puncture device 10 according to the embodiment described above can suppress vibration of the needle 17a. The controller 51 performs the first operation S1 in which the second driver 16 is moved in the puncture direction to puncture the cell S with the needle 17a, and the second operation S2 in which the second driver 16 is moved in the withdrawal direction opposite the puncture direction to reduce vibration occurring within the cell puncture device 10 due to the first operation S1. This reduces the vibration occurring within the cell puncture device 10 due to the first operation S1 by vibration occurring within the cell puncture device 10 due to the second operation S2, resulting in suppression of vibration of the needle 17a. Thus, for example, it is possible to suppress vibration of the needle tip inside the cell wall when the needle 17a punctures the cell S, thereby preventing damage to the interior of the cell wall. For example, it is also possible to prevent the needle 17a from being inserted into or removed from the cell S repeatedly.

In the present embodiment in which the first operation S1 is performed after the second operation S2, the amount of movement of the second driver 16 in the second operation S2 is greater than or equal to the amount of movement of the second driver 16 in the first operation S1. This allows the energy of vibration occurring within the cell puncture device 10 in the second operation S2 to be greater than the energy of vibration occurring within the cell puncture device 10 in the first operation S1. Therefore, even when vibration that has occurred within the cell puncture device 10 in the second operation S2 is somewhat attenuated between the second operation S2 and the first operation S1, the cell puncture device 10 can be maintained in a vibrating state with a certain level of energy. As a result, vibration occurring within the cell puncture device 10 due to the first operation S1 can be effectively reduced by vibration occurring within the cell puncture device 10 due to the second operation S2, thus effectively suppressing vibration of the needle 17a during the first operation S1.

Between the first operation S1 and the second operation S2, the controller 51 further performs the third operation S3 in which the second driver 16 is moved at a lower speed than in the first and second operations S1 and S2 in the puncture direction opposite the direction in the second operation S2. This allows the position of the second driver 16 that has been moved in the second operation S2 to be closer to its position prior to the second operation S2. As a result, the cell puncture device 10 facilitates performing the first operation S1 to be performed thereafter. In the third operation S3, the controller 51 moves the second driver 16 at a lower speed than in the second operation S2. Therefore, the third operation S3 is unlikely to affect the vibration that has occurred within the cell puncture device 10 due to the second operation S2. This prevents the third operation S3 from inhibiting the vibration suppression effect of the second operation S2.

The amount of movement of the second driver 16 in the third operation S3 is greater than or equal to the amount of movement of the second driver 16 in the second operation S2. This allows the position of the second driver 16 to be adjusted so that the amount of movement of the second driver 16 in the second operation S2 is greater than or equal to the amount of movement of the second driver 16 in the first operation S1. As a result, the energy of vibration occurring within the cell puncture device 10 during the second operation S2 can be greater than the energy of vibration occurring within the cell puncture device 10 during the first operation S1. Therefore, even when vibration that has occurred within the cell puncture device 10 during the second operation S2 is somewhat attenuated between the second operation S2 and the first operation S1, the cell puncture device 10 can be maintained in a vibrating state with a certain level of energy. Thereby, the vibration occurring within the cell puncture device 10 during the first operation S1 can be effectively reduced by the vibration occurring within the cell puncture device 10 during the second operation S2, thus effectively suppressing vibration of the needle 17a during the first operation S1.

The cell puncture device 10 has the base 12, and the arm 14 that is movably disposed with respect to the base 12 and supports the second driver 16. This allows the position of the second driver 16 relative to the base 12 to be easily adjusted by the arm 14. As a result, it becomes easy to adjust the position of the second driver 16 when the needle 17a punctures the cell S.

On the other hand, when the arm 14 is moved with respect to the base 12, the linear distance or path distance between the base 12 and the second driver 16 varies. When the linear distance or path distance between the base 12 and the second driver 16 varies, the period of the natural vibration of the arm 14 with respect to the base 12 varies. This is because variation in the position of the arm 14 causes variation in the rigidity of a portion constituting a region from the base 12 to the second driver 16 in the cell puncture device 10. From the viewpoint of vibration suppression, it is preferable to vary the time interval between the start time of the first operation S1 and the start time of the second operation S2 according to the variation in the period of the natural vibration of the arm 14. Specifically, when the second driver 16 is moved in the puncture direction in the first operation S1 and the second driver 16 is moved in the withdrawal direction in the second operation S2, as in the present embodiment, the time interval should theoretically be an integral multiple of the period of the natural vibration of the arm 14.

Based on the above, the controller 51 calculates the time interval between the start time of the first operation S1 and the start time of the second operation S2 according to the linear distance or path distance between the fixed part and the second driver 16 supported by the arm 14. This allows the cell puncture device 10 to perform the first operation S1 and the second operation S2 based on the calculated time interval, thus effectively suppressing vibration of the needle 17a. In the present embodiment, for example, the controller 51 may calculate the time interval as an integral multiple of the period of the natural vibration of the arm 14. Here, in the present disclosure, the period of the natural vibration of the arm 14 is not limited to the period of vibration of the arm 14 when only the arm 14 is vibrating, but may also include the period of vibration of the arm 14 when the arm 14 is vibrating together with other components of the cell puncture device 10. For example, the entirety of the first fixed part 11, the base 12, the first driver 13, and the arm 14 connected to the support 24 may deform and vibrate. The period of the natural vibration of the arm 14 may include, for example, the period of vibration of the arm 14 including deformation of the first fixed part 11 with respect to the support 24 when the deformation of the first fixed part 11 causes vibration to occur. The form of the cell puncture device 10 is not limited to that illustrated in FIG. 1. Therefore, the period of the natural vibration of the arm 14 may be the period of vibration of the arm 14 according to various forms of the cell puncture device 10.

The controller 51 may calculate the period of the natural vibration of the arm 14 from the vibration value of the arm 14 detected by the sensor unit 30, and calculate the time interval between the start time of the first operation S1 and the start time of the second operation S2 according to the period. Thus, the cell puncture device 10 can easily calculate the period of the natural vibration of the arm 14 using the sensor unit 30. Therefore, it also becomes easier for the cell puncture device 10 to calculate the time interval.

The controller 51 may calculate the time interval to satisfy the following condition: $0.9 \times m \times p \leq t \leq 1.1 \times m \times p$
m: an integer greater than or equal to 1,
p: the period of the natural vibration of the arm 14,
t: the time interval.
This causes the time interval to be closer to an integral multiple (m×p) of the period of the natural vibration of the arm 14, which is a theoretically preferred value, thus effectively suppressing vibration of the needle 17a.

The controller 51 moves the second driver 16 so that the movement speed of the needle 17a in the first operation S1 is 5 m/s or more. The cell puncture device 10 can thereby puncture the cell S with the needle 17a at a speed sufficient for the needle 17a to penetrate the cell wall, even when the cell S is a plant cell having a hard cell wall.

### (Variations)

While the present disclosure is described based on the drawings and embodiment, it should be noted that various modifications and revisions can be made by those skilled in the art based on the present disclosure. Accordingly, such modifications and revisions are included within the scope of the present disclosure. For example, the functions included in each configuration or each operation can be rearranged in a logically consistent manner, and multiple configurations or operations can be combined into one or divided.

For example, the shape, pattern, size, disposition, orientation, type, number, and the like of each component described above are not limited to those illustrated in the above description and drawing. The shape, pattern, size, disposition, orientation, type, number, and the like of each component may be configured arbitrarily, as long as the function can be realized. Each component of the cell puncture device 10 and the microscope system 1 illustrated in the drawing is a functional concept, and the specific form of each component is not limited to that illustrated in the drawing.

For example, a configuration that causes a general purpose electronic device such as a smartphone or a computer to function as the controller 51 of the cell puncture device 10 according to the above embodiment is possible. Specifically, a program in which processing for realizing the functions of the controller 51 of the cell puncture device 10 according to the embodiment is written may be stored in a memory of the electronic device, and the program may be read and executed by a processor of the electronic device. Accordingly, the present disclosure can also be implemented as a program executable by a processor.

Alternatively, the present disclosure can also be implemented as a non-transitory computer readable medium storing a program executable by a single processor or a plurality of processors to cause the cell puncture device 10 according to the embodiment to execute the functions. It is to be understood that these are also included within the scope of the present disclosure.

The above embodiment describes a configuration in which the controller 51 performs the second operation S2 and the first operation S1 in this order, but the present disclosure is not limited thereto. The controller 51 may perform the first operation S1 and the second operation S2 in this order. Even in such a case, vibration occurring within the cell puncture device 10 due to the first operation S1 can be reduced by vibration occurring within the cell puncture device 10 due to the second operation S2. As a result, it is possible to prevent vibration occurring within the cell puncture device 10 from affecting the subsequent operations of the cell puncture device 10.

The above embodiment describes a configuration in which in the second operation S2, the controller 51 moves the second driver 16 in the withdrawal direction opposite the puncture direction, to reduce vibration occurring within the cell puncture device 10 due to the first operation S1, but the present disclosure is not limited thereto. In the second operation S2, the controller 51 may move the second driver 16 in the puncture direction to reduce vibration occurring within the cell puncture device 10 due to the first operation S1.

The above embodiment describes a configuration in which when the controller 51 performs the first operation S1 after the second operation S2, the amount of movement of the second driver 16 in the second operation S2 is greater than or equal to the amount of movement of the second driver 16 in the first operation S1, but the present disclosure is not limited thereto. When the controller 51 performs the first operation S1 after the second operation S2, the amount of movement of the second driver 16 in the second operation S2 may be less than the amount of movement of the second driver 16 in the first operation S1. This allows the cell puncture device 10 to leave some vibration in the needle 17a during the first operation S1, by making the energy of vibration occurring within the cell puncture device 10 due to the second operation S2 less than the energy of vibration occurring within the cell puncture device 10 due to the first operation S1. As a result, it becomes easier for the cell puncture device 10 to puncture the cell S with the needle 17a in the first operation S1.

The above embodiment describes a configuration in which the controller 51 further performs the third operation S3 between the first operation S1 and the second operation S2, but the present disclosure is not limited thereto. The controller 51 may not perform the third operation S3.

The above embodiment describes a configuration in which the amount of movement of the second driver 16 in the third operation S3 is greater than or equal to the amount of movement of the second driver 16 in the preceding operation (second operation S2 in the above embodiment) performed before the third operation S3, but the present disclosure is not limited thereto. The amount of movement of the second driver 16 in the third operation S3 may be less than the amount of movement of the second driver 16 in the preceding operation. This allows the cell puncture device 10 to leave some vibration in the needle 17a during the subsequent operation by making the energy of vibration occurring within the cell puncture device 10 due to the preceding operation less than the energy of vibration occurring within the cell puncture device 10 due to the subsequent operation performed after the third operation S3. As a result, it becomes possible for the cell puncture device 10 to easily puncture the cell S with the needle 17a, for example, when the preceding operation is the second operation S2 and the subsequent process is the first operation S1.

The above embodiment describes a configuration in which the cell puncture device 10 includes the base 12 and the arm 14 that is movably disposed with respect to the base 12 and supports the second driver 16, but the present disclosure is not limited thereto. The cell puncture device 10 may not include the base 12 and the arm 14. In this case, the cell puncture device 10 may be used by being mounted on an external component, such as a movable trestle.

The above embodiment describes a configuration in which the controller 51 calculates the time interval between the start time of the first operation S1 and the start time of the second operation S2 according to the linear distance or path distance between the base 12 and the second driver 16, but the present disclosure is not limited thereto. For example, the controller 51 may calculate the time interval between the start time of the first operation S1 and the start time of the second operation S2 according to the linear distance or path distance between the first fixed part 11 and the second driver 16. When variation in the linear distance or path distance between the base 12 and the second driver 16 is small, for example, when the movement range of the arm 14 relative to the base 12 is limited, the controller 51 may not perform processing of calculating the time interval according to the linear distance or path distance between the base 12 and the second driver 16.

The above embodiment describes a configuration in which the controller 51 may calculate the period of the natural vibration of the arm 14 from the vibration value of the arm 14 detected by the sensor unit 30, and calculate the time interval between the start time of the first operation S1 and the start time of the second operation S2 according to the period. The above embodiment describes a configuration in which the controller 51 may calculate the time interval using the reference values for the time interval stored in advance in the memory 53 or the like. However, a method for calculating the time interval is not limited thereto. The controller 51 may calculate the time interval using a calculation method other than those described in the above embodiment.

In both the first operation S1 and the second operation S2, when the second driver 16 is moved in the puncture direction, the controller 51 may calculate the time interval so as to satisfy the following condition: $0.9 \times \left(2m-1\right) \times p / 2 \leq t \leq 1.1 \times \left(2m-1\right) \times p / 2$
m: an integer greater than 1,
p: the period of the natural vibration of the arm 14,
t: the time interval.
This causes the time interval to be closer to a theoretically preferred value ((2m-1)×p/2), thus effectively suppressing vibration of the needle 17a.

The above embodiment describes a configuration in which the controller 51 moves the second driver 16 so that the movement speed of the needle 17a in the first operation S1 is 5 m/s or more, but the present disclosure is not limited thereto. The controller 51 may move the second driver 16 so that the movement speed of the needle 17a in the first operation S1 is less than 5 m/s.

### EXAMPLES

The cell puncture device 10 according to the present embodiment will be described in more detail below using examples, but the present disclosure is not limited to the following examples. The numerical values described in the examples are just examples and do not limit the scope of the present disclosure. The scope of the present disclosure should be defined solely on the basis of the claims. In the following description, the same components as in the embodiment are denoted by the same reference numerals, and redundant descriptions are omitted.

In the examples, the vibration state of the cell puncture device 10 was simulated using numerical calculations to verify the effectiveness of the cell puncture device 10 of the present embodiment. The following is a description of a simulation method.

For example, the vibration state of the arm 14 can be expressed by the following equation of motion: $f \left(t\right) - {kx}_{a} - c {\dot{x}}_{a} = m {{x}^{¨}}_{a}$ wherein ma represents the mass of the arm 14, spring constant ka represents the stiffness of the arm 14, ca represents the attenuation coefficient of the arm 14, fa(t) represents a force applied to the arm 14, and xa represents the position of the arm 14. In the examples, not only the arm 14, but also the first driver 13, the base 12, and the first fixed part 11 were also mathematized and simulated.

An excitation force due to the movement of the second driver 16 can be expressed by the following equation: $P = {\int }_{O}^{t} Fdt = mΔV$ wherein when the second driver 16 is moved, P represents an impulse generated by the movement of the second driver 16 during a minute time interval t, F represents an impact force, ΔV represents a velocity change during the movement, and m represents the mass of a portion moved by the movement of the second driver 16.

In the cell puncture device 10, the amount of movement of the movable part 16b by the piezoelectric element 16c is detected by a sensor, and PID-based feedback control is applied to the movement of the second driver 16 by the piezoelectric element 16c. This control can be mathematized using Laplace transform or the like. In the examples, in addition to PID control, filters such as low-pass and/or notch filters were applied before and after the PID control for the purpose of stabilizing the control, and these filters were also mathematized and simulated.

These equations can also be simulated numerically by the finite difference method or other methods. The numerical simulations were also performed in the examples.

### (Comparative Example)

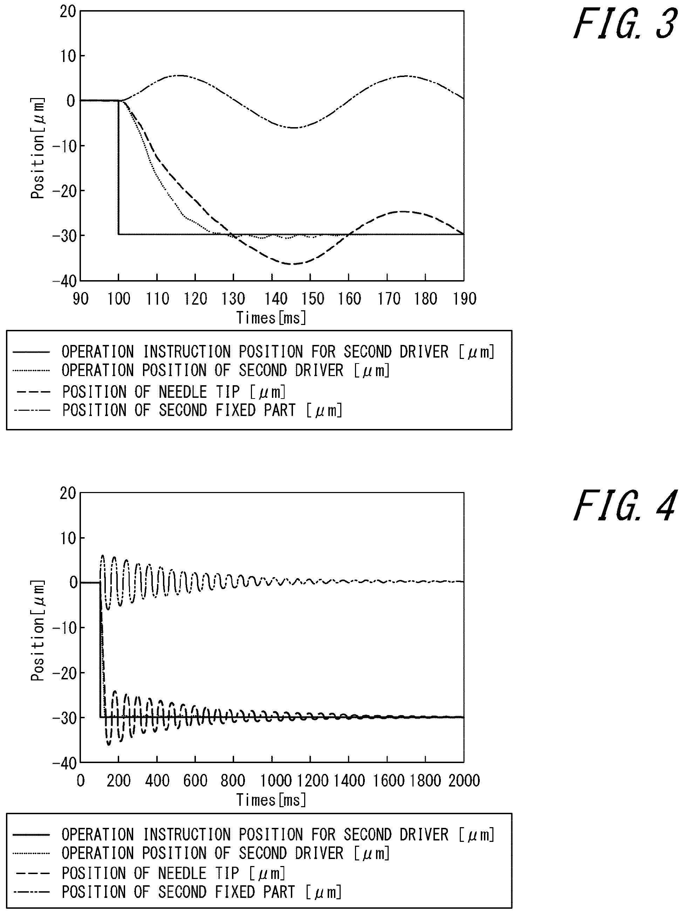

As a comparative example, the results of simulating the vibration state of a conventional cell puncture device are described. FIGS. 3 and 4 are graphs of the simulated vibration state of the conventional cell puncture device. Specifically, FIGS. 3 and 4 illustrate variations in the position of each part of the cell puncture device and the position of a needle tip over time when a controller has performed only a first operation. FIG. 3 illustrates an enlarged view of the variations over time for a short period (0 ms to about 200 ms), while FIG. 4 illustrates the variations over time for a longer period (0 ms to about 2000 ms).

**In** the comparative example, the controller first instructs a second driver to move in a puncture direction (see solid lines in FIGS. 3 and 4). In accordance with this instruction, the second driver is moved to an operation position of the second driver (see the dotted lines in FIGS. 3 and 4) with a delay in the instruction from the controller to the second driver. Here, the dotted lines in FIGS. 3 and 4, which indicate the operation position of the second driver, indicate the relative position of the movable part to the second fixed part, not the absolute position of the movable part of the second driver.

This delayed response is caused by the weight of a movable part of the second driver, and the weights of a support head fixing part and a needle unit connected to the movable part. Specifically, even when a force is exerted to rapidly move the second driver to an operation instruction position by expansion or contraction of a piezoelectric element, the second driver cannot be moved immediately due to inertia caused by the weights mentioned above. This results in the delayed response.

Here, for example, the second driver may include an operation sensor that measures the operation of the second driver. In this case, the amount of movement of the second driver can be controlled by providing feedback with the operation sensor. PID or PI control can be adopted as the feedback control method. The example illustrated in FIGS. 3 and 4 uses a PI-based feedback control method.

On the other hand, the second fixed part of the second driver receives a reaction force on the opposite side (withdrawal direction) to the direction (puncture direction) in which the movable part of the second driver is moved due to the operation of the second driver. Therefore, as illustrated in FIGS. 3 and 4, the movement of the second driver in the puncture direction causes the second fixed part of the second driver to move in the withdrawal direction. The second fixed part of the second driver is coupled to the arm, so the elastic force of the arm generates a force in the opposite direction to the direction in which the second fixed part is moved. As a result, as illustrated in the chain double-dashed lines in FIGS. 3 and 4, the second fixed part vibrates at the period of natural vibration of the arm. This vibration is attenuated by the viscosity of the arm and its surrounding materials, but in this comparative example, the vibration remains for a long time.

As illustrated in FIGS. 3 and 4, the absolute position of the needle (dashed line) is the sum of the position of the second fixed part (chain double-dashed line) and the operation position of the second driver (dotted line). In this comparative example, the needle significantly vibrates immediately after the operation of the second driver. Thereafter, the vibration of the needle is gradually attenuated, but in this comparative example, the vibration remains for a long time. In other words, the use of a cell puncture device that performs control as in this comparative example causes a needle to puncture a cell repeatedly.

### (Summary of Examples 1 to 8)

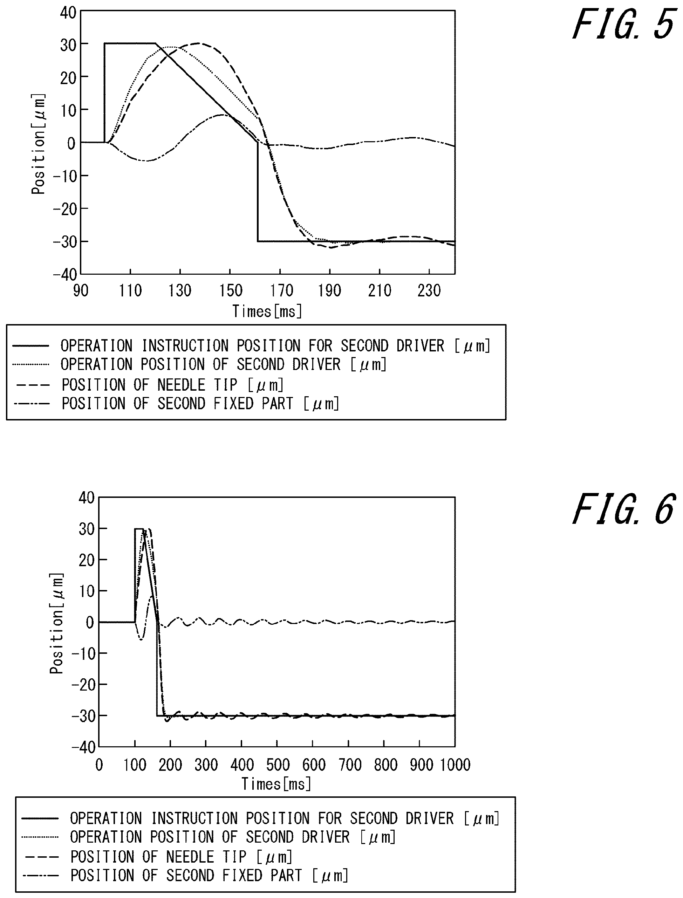

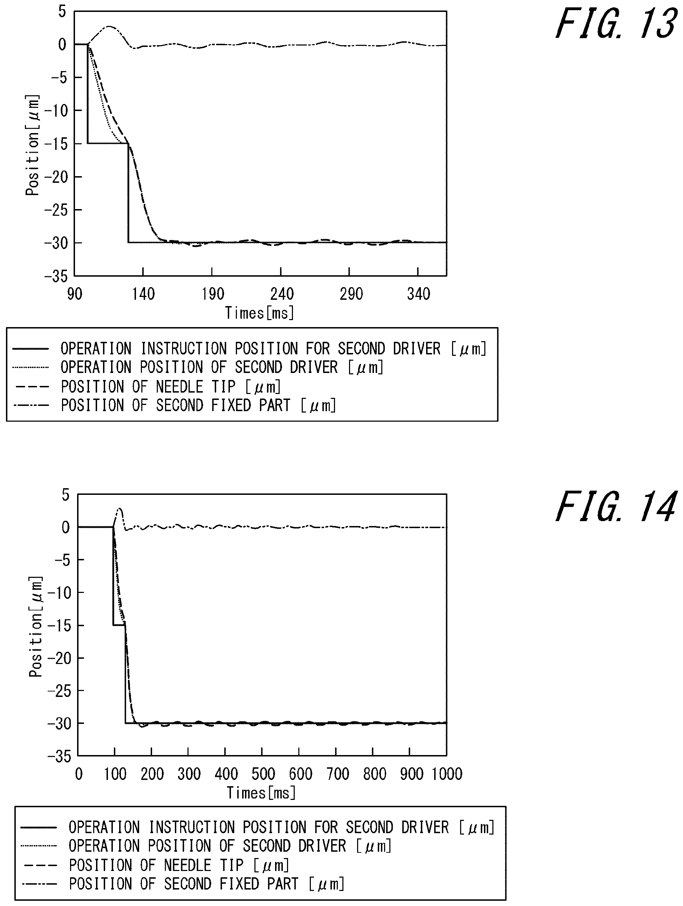

In the first to eighth examples, the vibration state of the cell puncture device 10 was simulated. Specifically, in the first to fourth examples (see FIGS. 5 to 12), variations in the position of each part of the cell puncture device 10 and the position of the needle tip over time were simulated when the second driver 16 was moved in the withdrawal direction in the second operation S2. In the fifth to eighth examples (see FIGS. 13 to 18, not illustrated for the eighth example), variations in the position of each part of the cell puncture device 10 and the position of the needle tip over time were simulated when the second driver 16 was moved in the puncture direction in the second operation S2. In the fifth to eighth examples, the third operation S3 was omitted as the result of the second driver 16 moving in the puncture direction in the second operation S2. As illustrated in FIGS. 5 to 18, for the individual examples, graphs illustrating the variations over time in a short period (0 ms to about 200 ms) in an enlarged manner (specifically, FIGS. 5, 7, 9, 11, 13, 15, and 17) and graphs illustrating the variations over time in a long period (0 ms to about 1000 ms) (specifically, FIGS. 6, 8, 10, 12, 14, 16, and 18) were generated. In FIGS. 5 to 18, the positive direction of the vertical axis corresponds to the withdrawal direction, and the negative direction of the vertical axis corresponds to the puncture direction.

### (First Example)

As illustrated in FIGS. 5 and 6, in the first example, the second operation S2 in which the second driver 16 was instructed to move in the withdrawal direction opposite the puncture direction was first performed. In the first example, the amount of movement of the second driver 16 in the second operation S2 was the same as that of the second driver 16 in the first operation S1. This allows a similar force to a reaction force generated in the first operation S1 to be generated in the second operation S2.

Next, the third operation S3 was performed to return the position of the second driver 16 to its position prior to the second operation S2. As illustrated in FIGS. 5 and 6, in the third operation S3, in order to reduce the generation of a reaction force associated with movement, the movement was instructed to vary the position in an inclined manner so that the position was not varied abruptly.

Next, the first operation S1 was performed. In the first example, the time interval between the start time of the second operation S2 and the start time of the first operation S1 was set to be the same as one period of the natural vibration of the arm 14. The second driver 16 was thereby moved in the puncture direction at the timing when the arm 14 was moved in the puncture direction, and the arm 14 was subjected to a reaction force in the withdrawal direction opposite the puncture direction. The arm 14 was prevented from moving in the puncture direction, thus suppressing vibration of the arm 14.

As illustrated in FIGS. 5 and 6, in the first example, vibration of the needle 17a occurred immediately after the second operation S2 was performed. However, it was confirmed that the vibration was significantly reduced in a time region after the first period of the natural vibration of the arm 14. It was also confirmed that the vibration of the needle 17a after the second operation S2 was performed was attenuated to a negligible level in a shorter period than in the comparative example.

### (Second Example)

The second example differs from the first example in the time interval between the start time of the second operation S2 and the start time of the first operation S1, while the other conditions are the same. Therefore, in the second example, the differences from the first example are mainly described.

In the second example, the time interval between the start time of the second operation S2 and the start time of the first operation S1 was set to be the same as two periods of the natural vibration of the arm 14. As in the first example, the second driver 16 was thereby moved in the puncture direction at the timing when the arm 14 was moved in the puncture direction, and the arm 14 was subjected to a reaction force in the withdrawal direction opposite the puncture direction. The arm 14 was prevented from moving in the puncture direction, thus suppressing vibration of the arm 14.

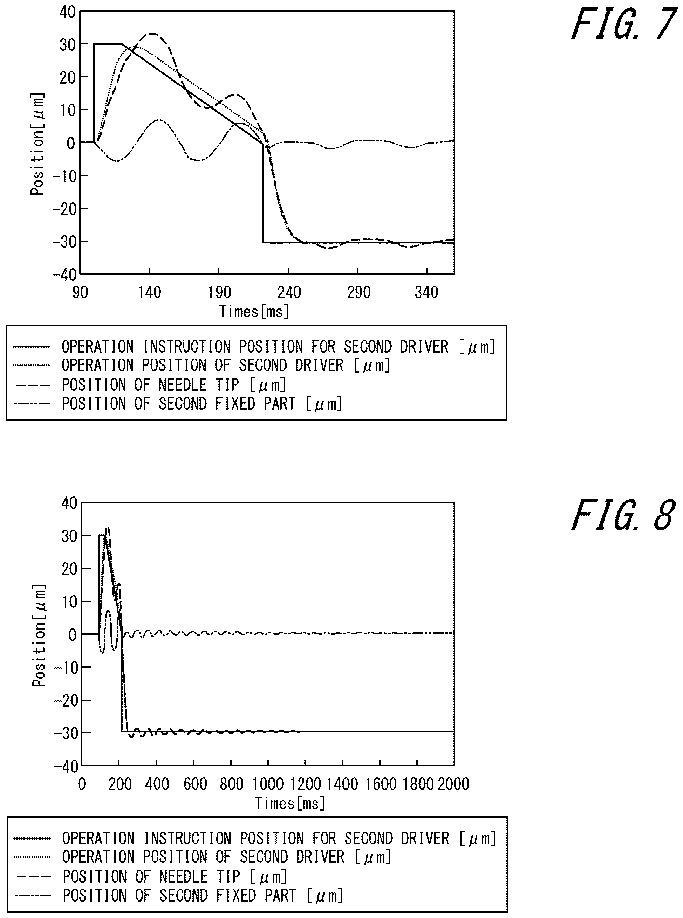

As illustrated in FIGS. 7 and 8, also in the second example, vibration of the needle 17a occurred immediately after the second operation S2 was performed. However, it was confirmed that the vibration was significantly reduced in a time region after the second period of the natural vibration of the arm 14. It was also confirmed that the vibration of the needle 17a after the second operation S2 was performed was attenuated to a negligible level in a shorter period than in the comparative example.

According to the first and second examples, it was confirmed that when the second driver 16 is moved in the puncture direction in the first operation S1 and the second driver 16 is moved in the withdrawal direction in the second operation S2, the vibration of the needle 17a can be effectively suppressed by setting the time interval to approximately an integral multiple of the period of the natural vibration of the arm 14.

### (Third Example)

The third example differs from the first example in that the amount of movement of the second driver 16 in the withdrawal direction in the second operation S2 is less than the amount of movement of the second driver 16 in the puncture direction in the first operation S1, while the other conditions are the same. Therefore, in the third example, the differences from the first example are mainly described.

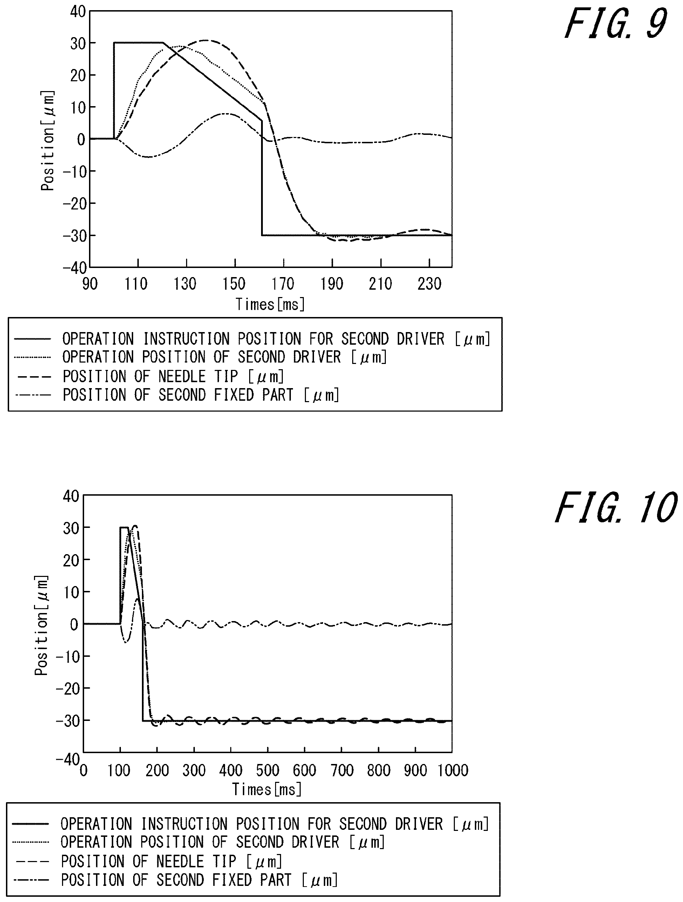

As illustrated in FIGS. 9 and 10, in the third example, the amount of movement of the second driver 16 in the withdrawal direction in the second operation S2 was less than the amount of movement of the second driver 16 in the puncture direction in the first operation S1.

As illustrated in FIGS. 9 and 10, also in the third example, vibration of the needle 17a occurred immediately after the second operation S2 was performed. However, it was confirmed that the vibration was significantly reduced in a time region after the first period of the natural vibration of the arm 14. It was also confirmed that the vibration of the needle 17a after the second operation S2 was performed was attenuated to a negligible level in a shorter period than in the comparative example.

In the third example, the degree to which the vibration of the needle 17a is reduced by the second operation S2 differs from that in the first example. In other words, it was confirmed that by varying the ratio between the amount of movement of the second driver 16 in the second operation S2 and the amount of movement of the second driver 16 in the first operation S1, the ratio between the amount of vibration occurring during the second operation S2 and the amount of vibration occurring during the first operation S1 can be varied, which allows residual vibration of the needle 17a to be reduced or allows a certain amount of residual vibration to remain in the needle 17a.

### (Fourth Example)

The fourth example differs from the first example in that the amount of movement of the second driver 16 in the withdrawal direction in the second operation S2 is greater than the amount of movement of the second driver 16 in the puncture direction in the first operation S1, while the other conditions are the same. Therefore, in the fourth example, the differences from the first example are mainly described.

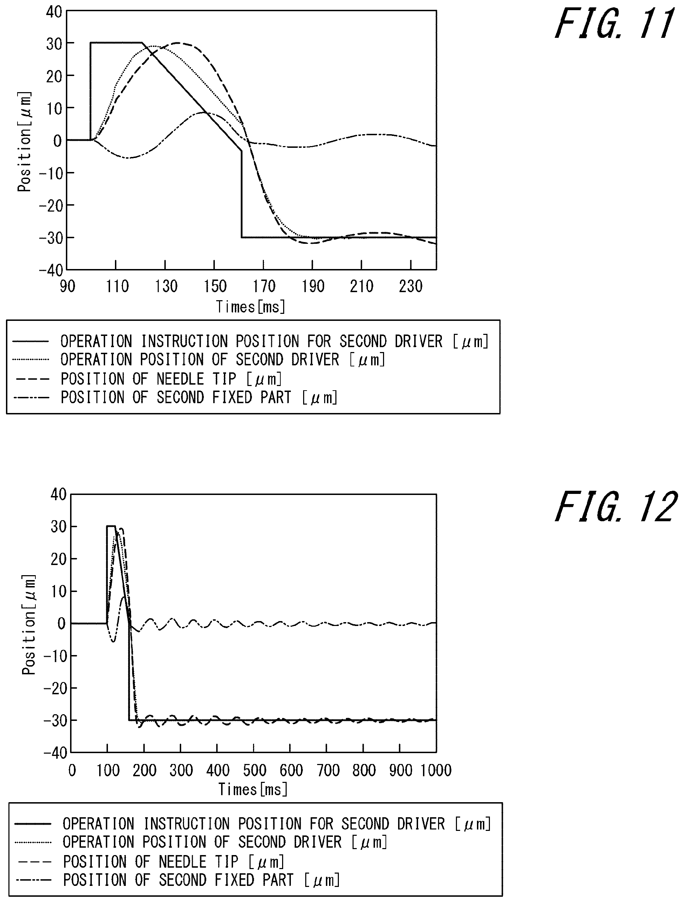

As illustrated in FIGS. 11 and 12, in the fourth example, the amount of movement of the second driver 16 in the withdrawal direction in the second operation S2 was greater than the amount of movement of the second driver 16 in the puncture direction in the first operation S1.

As illustrated in FIGS. 11 and 12, also in the fourth example, vibration of the needle 17a occurred immediately after the second operation S2 was performed. However, it was confirmed that the vibration was significantly reduced in a time region after the first period of the natural vibration of the arm 14. It was also confirmed that the vibration of the needle 17a after the second operation S2 was performed was attenuated to a negligible level in a shorter period than in the comparative example.

In the fourth example, the degree to which the vibration of the needle 17a is reduced by the second operation S2 differs from that in the first example. In other words, it was confirmed that by varying the ratio between the amount of movement of the second driver 16 in the second operation S2 and the amount of movement of the second driver 16 in the first operation S1, the ratio between the amount of vibration occurring during the second operation S2 and the amount of vibration occurring during the first operation S1 can be varied, which allows residual vibration of the needle 17a to be reduced or allows a certain amount of residual vibration to remain in the needle 17a.

In particular, since the vibration that has occurred in the second operation S2 attenuates and becomes smaller by the time of reaching the first operation S1, it is conceived that vibration of the needle 17a can be reduced more effectively by making the amount of movement of the second driver 16 in the second operation S2 greater than the amount of movement of the second driver 16 in the first operation S1.

### (Fifth Example)

The fifth example differs from the first example in that the second driver 16 is moved in the puncture direction in both of the first operation S1 and the second operation S2, and the third operation S3 is not performed, while the other conditions are the same. Therefore, in the fifth example, the differences from the first example are mainly described.

As illustrated in FIGS. 13 and 14, in the fifth example, the second driver 16 was moved in the puncture direction in both of the first operation S1 and the second operation S2, and the third operation S3 was not performed. As illustrated in FIGS. 13 and 14, in the fifth example, the time interval between the start time of the second operation S2 and the start time of the first operation S1 was set to be the same as 1/2 periods of the natural vibration of the arm 14.

As illustrated in FIGS. 13 and 14, also in the fifth example, vibration of the needle 17a occurred immediately after the second operation S2 was performed. However, it was confirmed that the vibration was significantly reduced in a time region after the 1/2 period of the natural vibration of the arm 14. It was also confirmed that the vibration of the needle 17a after the second operation S2 was performed was attenuated to a negligible level in a shorter period than in the comparative example.

### (Sixth Example)

The sixth example differs from the fifth example in the time interval between the start time of the second operation S2 and the start time of the first operation S1, while the other conditions are the same. Therefore, in the sixth example, the differences from the fifth example are mainly described.

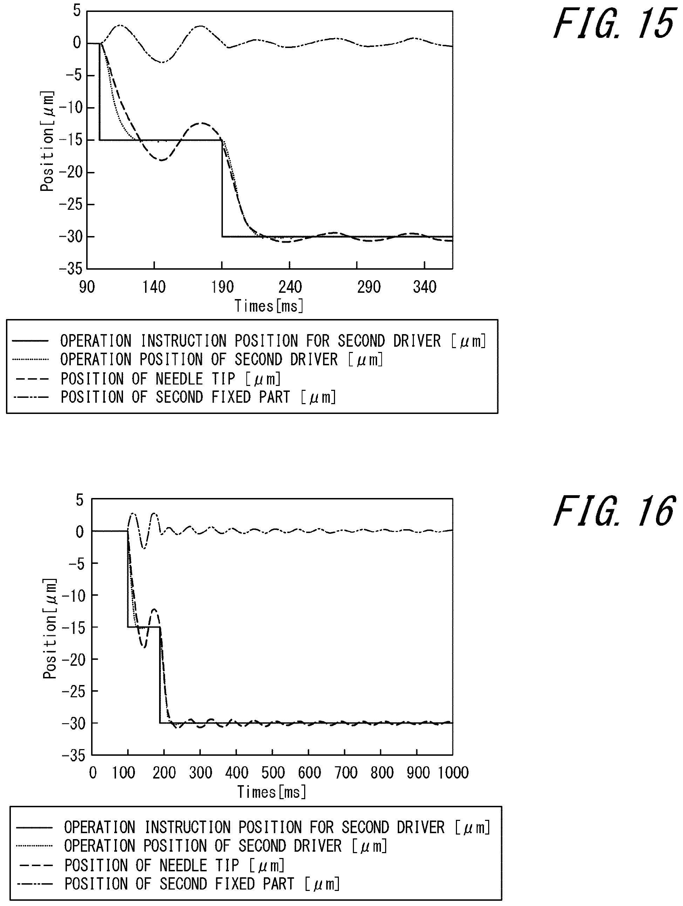

As illustrated in FIGS. 15 and 16, in the sixth example, the time interval between the start time of the second operation S2 and the start time of the first operation S1 was set to be the same as 3/2 periods of the natural vibration of the arm 14.

As illustrated in FIGS. 15 and 16, also in the sixth example, vibration of the needle 17a occurred immediately after the second operation S2 was performed. However, it was confirmed that the vibration was significantly reduced in a time region after the 3/2 period of the natural vibration of the arm 14. It was also confirmed that the vibration of the needle 17a after the second operation S2 was performed was attenuated to a negligible level in a shorter period than in the comparative example.

According to the fifth and sixth examples, it was confirmed that when the second driver 16 is moved in the puncture direction in both of the first operation S1 and the second operation S2, the vibration of the needle 17a can be effectively suppressed by setting the time interval to approximately 1/2 of the period of the natural vibration of the arm 14 + an integral multiple of the period of the natural vibration of the arm 14. When the time interval is set to 1/2 of the period of the natural vibration as in the fifth example, the interval between the first operation S1 and the second operation S2 may be too short to allow the third operation S3 to operate. In such a case, the control to lengthen the time interval as in the sixth example is effective.

### (Seventh Example)

The seventh example differs from the sixth example in that the amount of movement of the second driver 16 in the puncture direction in the second operation S2 is greater than the amount of movement of the second driver 16 in the puncture direction in the first operation S1, while the other conditions are the same. Therefore, in the seventh example, the differences from the sixth example are mainly described.

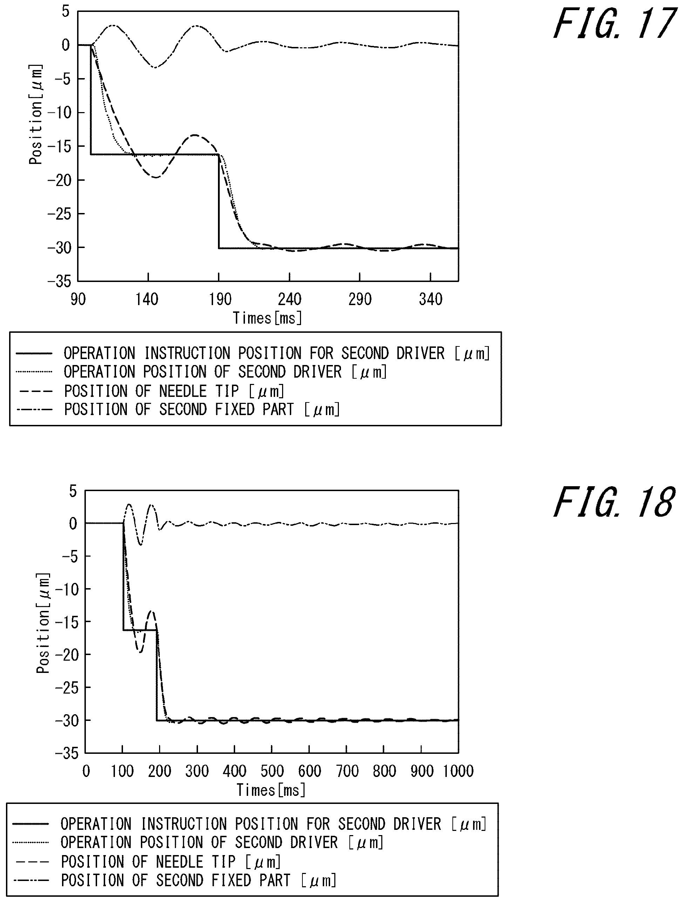

As illustrated in FIGS. 17 and 18, in the seventh example, the amount of movement of the second driver 16 in the puncture direction in the second operation S2 was greater than the amount of movement of the second driver 16 in the puncture direction in the first operation S1.

As illustrated in FIGS. 17 and 18, also in the seventh example, vibration of the needle 17a occurred immediately after the second operation S2 was performed. However, it was confirmed that the vibration was significantly reduced in a time region after the 3/2 period of the natural vibration of the arm 14. It was also confirmed that the vibration of the needle 17a after the second operation S2 was performed was attenuated to a negligible level in a shorter period than in the comparative example.

In the seventh example, the degree to which the vibration of the needle 17a is reduced by the second operation S2 differs from that in the sixth example. In other words, it was confirmed that by varying the ratio between the amount of movement of the second driver 16 in the second operation S2 and the amount of movement of the second driver 16 in the first operation S1, the ratio between the amount of vibration occurring during the second operation S2 and the amount of vibration occurring during the first operation S1 can be varied, which allows residual vibration of the needle 17a to be reduced or allows a certain amount of residual vibration to remain in the needle 17a.

In particular, since the vibration that has occurred in the second operation S2 attenuates and becomes smaller by the time of reaching the first operation S1, it is conceived that vibration of the needle 17a can be reduced more effectively by making the amount of movement of the second driver 16 in the second operation S2 greater than the amount of movement of the second driver 16 in the first operation S1.

### (Eighth Example)

The eighth example (not illustrated) differs from the sixth example in that the amount of movement of the second driver 16 in the puncture direction in the second operation S2 is less than the amount of movement of the second driver 16 in the puncture direction in the first operation S1, while the other conditions are the same. Therefore, in the eighth example, the differences from the sixth example are mainly described.

In the eighth example, the amount of movement of the second driver 16 in the puncture direction in the second operation S2 was less than the amount of movement of the second driver 16 in the puncture direction in the first operation S1. As a result, also in the eighth example, vibration of the needle 17a occurred immediately after the second operation S2 was performed. However, it was confirmed that the vibration was significantly reduced in a time region after the 3/2 period of the natural vibration of the arm 14. It was also confirmed that the vibration of the needle 17a after the second operation S2 was performed was attenuated to a negligible level in a shorter period than in the comparative example.

In the eighth example, it was confirmed that the degree to which the vibration of the needle 17a is reduced by the second operation S2 differs from that in the sixth example. In other words, it was confirmed that by varying the ratio between the amount of movement of the second driver 16 in the second operation S2 and the amount of movement of the second driver 16 in the first operation S1, the ratio between the amount of vibration occurring during the second operation S2 and the amount of vibration occurring during the first operation S1 can be varied, which allows residual vibration of the needle 17a to be reduced or allows a certain amount of residual vibration to remain in the needle 17a.

As described above, it was confirmed from the first to eighth examples that the cell puncture device 10, cell puncture method, and non-transitory computer readable medium according to the present embodiment can suppress vibration of the needle 17a.

In the examples described above, step inputs are applied by which the operation instruction amounts for the second driver 16 vary instantaneously. However, the present disclosure is not limited thereto, and ramp inputs may be applied by which the positions vary gradually over time. In such a case, the impulse generated by the reaction force produced by a ramp input in the second operation S2 can be made the same as that generated by the reaction force in the first operation S1. Alternatively, considering the amount of vibration energy that is dissipated by attenuation between the second operation S2 and the first operation S1, the amount of movement of the second driver 16 in the second operation S2 can be made greater than the amount of movement of the second driver 16 in the first operation S1.

## Claims

1. A cell puncture device, comprising:
a needle configured to puncture a cell along a puncture direction;
a driver configured to drive the needle; and
a controller configured to control operations of the driver,
wherein the controller is configured to perform:
a first operation in which the driver is moved in the puncture direction to puncture the cell with the needle; and
a second operation in which the driver is moved in the puncture direction or in a withdrawal direction opposite the puncture direction to reduce vibration occurring within the cell puncture device due to the first operation.

2. The cell puncture device according to claim 1, wherein the controller performs the second operation before the first operation.

3. The cell puncture device according to claim 2, wherein an amount of movement of the driver in the second operation is greater than or equal to an amount of movement of the driver in the first operation.

4. The cell puncture device according to claim 2, wherein an amount of movement of the driver in the second operation is less than an amount of movement of the driver in the first operation.

5. The cell puncture device according to any one of claims 1 to 4, wherein when the driver is moved in the withdrawal direction in the second operation, the controller is configured to further perform, between the first operation and the second operation, a third operation in which the driver is moved in an opposite direction from a preceding operation at a lower speed than in the first and second operations.

6. The cell puncture device according to claim 5, wherein an amount of movement of the driver in the third operation is greater than or equal to an amount of movement of the driver in the preceding operation, among the first and second operations, that is performed before the third operation.

7. The cell puncture device according to any one of claims 1 to 4, comprising:
a base; and
an arm disposed movably with respect to the base, the arm being configured to support the driver.

8. The cell puncture device according to claim 7, wherein the controller is configured to calculate a time interval between a start time of the first operation and a start time of the second operation, according to a linear distance or a path distance between the base and the driver supported by the arm.

9. The cell puncture device according to claim 7, comprising a fixed part disposed with respect to an imager configured to image the cell,
wherein the controller is configured to calculate a time interval between a start time of the first operation and a start time of the second operation, according to a linear distance or a path distance between the fixed part and the driver supported by the arm.

10. The cell puncture device according to claim 7, comprising a sensor unit disposed on the arm, the sensor unit being configured to detect a vibration state of the arm,
wherein the controller is configured to calculate a period of natural vibration of the arm from a vibration value of the arm detected by the sensor unit, and calculate a time interval between a start time of the first operation and a start time of the second operation according to the period.

11. The cell puncture device according to claim 8, wherein
when the driver is moved in the withdrawal direction in the second operation, a following condition is satisfied: $0.9 \times m \times p \leq t \leq 1.1 \times m \times p$
m: an integer greater than or equal to 1,
p: a period of natural vibration of the arm,
t: the time interval, and
when the driver is moved in the puncture direction in the second operation, a following condition is satisfied: $0.9 \times \left(2m-1\right) \times p / 2 \leq t \leq 1.1 \times \left(2m-1\right) \times p / 2$
m: an integer greater than or equal to 1,
p: a period of natural vibration of the arm,
t: the time interval.

12. The cell puncture device according to claim 10, wherein the period of the natural vibration of the arm is a period of the arm when only the arm is vibrating, or a period of the arm when the arm is vibrating together with another component of the cell puncture device.

13. The cell puncture device according to any one of claims 1 to 4, wherein the controller is configured to move the driver such that a movement speed of the needle in the first operation is 5 m/s or more.

14. A cell puncture method using a cell puncture device including a needle configured to puncture a cell along a puncture direction, and a driver configured to drive the needle, the cell puncture method comprising:
a first operation in which the driver is moved in the puncture direction to puncture the cell with the needle, and
a second operation in which the driver is moved in the puncture direction or in a withdrawal direction opposite the puncture direction to reduce vibration occurring within the cell puncture device due to the first operation.

15. A non-transitory computer readable medium storing a program executable by one or more processors, the program configured to cause a cell puncture device to execute operations, the cell puncture device including a needle configured to puncture a cell along a puncture direction, and a driver configured to drive the needle, the operations comprising:
a first operation in which the driver is moved in the puncture direction to puncture the cell with the needle, and
a second operation in which the driver is moved in the puncture direction or in a withdrawal direction opposite the puncture direction to reduce vibration occurring within the cell puncture device due to the first operation.
